(19) Europäisches Patentamt
European Patent Office
Office européen des brevets

(11) EP 2 552 394 B1

(12) **EUROPÄISCHE PATENTSCHRIFT**

(45) Veröffentlichungstag und Bekanntmachung des Hinweises auf die Patenterteilung:
**02.11.2016 Patentblatt 2016/44**

(21) Anmeldenummer: **11702597.3**

(22) Anmeldetag: **28.01.2011**

(51) Int Cl.:
***A61K 8/49*** *(2006.01)* ***A61Q 5/10*** *(2006.01)*
***A61K 8/31*** *(2006.01)*

(86) Internationale Anmeldenummer:
**PCT/EP2011/051200**

(87) Internationale Veröffentlichungsnummer:
**WO 2011/124402 (13.10.2011 Gazette 2011/41)**

(54) **FÄRBEMITTEL MIT LIPOPHILEM ACYLPYRIDINIUM-KOMPLEX**

DYE WITH LIPOPHILIC ACYLPYRIDINIUM COMPLEX

AGENT COLORANT COMPRENANT UN COMPLEXE D'ACYLPYRIDINIUM LIPOPHILE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.03.2010 DE 102010003395**

(43) Veröffentlichungstag der Anmeldung:
**06.02.2013 Patentblatt 2013/06**

(73) Patentinhaber: **Henkel AG & Co. KGaA**
**40589 Düsseldorf (DE)**

(72) Erfinder:
- **MANNECK, Hartmut**
  **23860 Klein Wesenberg (DE)**
- **KLEEN, Astrid**
  **20457 Hamburg (DE)**
- **BREWER, Marie-Lisa**
  **22761 Hamburg (DE)**

(56) Entgegenhaltungen:
**EP-A2- 2 295 027**
**WO-A1-2005/055966**
**WO-A1-2009/135700**
**WO-A1-2010/022996**
**WO-A2-2010/054981**
**US-B1- 6 371 993**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Mittel zum Färben von keratinischen Fasern, insbesondere menschlichen Haaren, enthaltend einen lipophilen Acylpyridinium-Komplex aus kationischen Acylpyridiniumderivate und Paraffinöl.

Die Veränderung von Form und Farbe der Haare stellt einen wichtigen Bereich der modernen Kosmetik dar. Zur Bereitstellung farbverändernder kosmetischer Mittel, insbesondere für keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten Oxidationsfarbstoffvorprodukte, unterteilt in sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich in der Regel durch lang anhaltende Färbeergebnisse aus. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen. Diese beiden Färbesysteme können zur Erzielung spezifischer Nuancen auch miteinander kombiniert werden.

Neben der Erzeugung von Naturnuancen, welche zumeist bei der dezenten Kaschierung von grauen Haaren Anwendung finden, ist die Ausbildung von modischen Nuancen ein Hauptanwendungsgebiet der oxidativen Haarfarben (Oxidationsfärbemittel). Insbesondere intensive Rot-, Rotbraun- und Kupfernuancen liegen bei der modischen Farbpalette im Fokus. Bei leuchtenden Modetönen ist daher insbesondere eine gute Farbintensität der Färbungen gefragt.

Es ist daher die Aufgabe der vorliegenden Erfindung, Mittel zur Färbung keratinischer Fasern zur Verfügung zu stellen, welche neben den üblichen Anforderungen einer Haarfarbe im Hinblick auf guter Grauabdeckung, gutem Egalisiervermögen und guten Echtheitseigenschaften gegenüber Haarwäschen, Schweiß, Wettereinflüssen und anderen Haarbehandlungen über eine verbesserte Farbintensität verfügen.

In überraschender Weise wurde nun gefunden, dass die Farbintensität von oxidativen Färbungen durch Mittel, die neben Oxidationsmitteln und Farbstoffen oder Farbstoffvorprodukten mindestens ein spezielles, kationisches Acylpyridiniumderivat in Kombination mit mindestens 5 Gew.-%, bezogen auf das Gesamtgewicht des Mittels, Paraffinöl enthalten, entscheidend verbessert werden kann. Die Substanzklasse der erfindungsgemäßen Acylpyridiniumderivate ist aus der Literatur bereits als Mittel zur Erzeugung von nichtoxidativen Färbungen auf Haaren (DE 197 45 356) oder aus Mitteln zur Aufhellung von Haaren (DE 10 2007 047685) bekannt. Der Einsatz von kationischen Acylpyridiniumderivaten in Aufhellmitteln in Kombination mit bestimmten Coaktivatoren ist aus der WO2009/135700 A1 bekannt. US 6371993B1 offenbart nicht-oxidative Färbemittel, die a) mindestens ein kationisches Acylpyridiniumderivat, das u. a ausgewählt sein kann aus Verbindungen der vorliegend beanspruchten Formel (I), und b) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, $\alpha$- bis $\omega$-Aminosäuren, aus 2 bis 9 Aminosäuren aufgebauten Oligopeptiden und aromatischen Hydroxyverbindungen, und/oder mindestens einer CH-aktiven Verbindung, enthalten, die intensive Ausfärbungen auch ohne Wasserstoffperoxid ermöglichen. Aus diesen Dokumenten ist für den Fachmann jedoch keinerlei Hinweis auf Verbesserungen der Farbintensität von oxidativen Färbungen durch solche Acylpyridiniumderivate in Kombination mit mindestens 5 Gew.-% Paraffinöl zu entnehmen.

Ein erster Gegenstand der Erfindung ist daher ein Mittel zum Färben von keratinischen Fasern, welches dadurch gekennzeichnet ist, dass es in einem kosmetischen Träger

(i) mindestens ein kationisches Acylpyridiniumderivat der Formel (I),

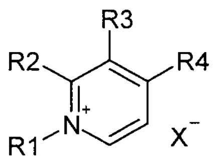

(I),

worin

| | |
|---|---|
| R1 | für eine $C_1$-$C_6$-Alkylgruppe, eine $C_2$-$C_6$-Alkenylgruppe, eine $C_2$-$C_6$-Hydroxy-alkylgruppe, eine $C_1$-$C_6$-Alkoxy-$C_2$-$C_6$-alkylgruppe, eine Carboxy-$C_2$-$C_6$-alkyl-gruppe, eine Aryl-$C_1$-$C_6$-alkylgruppe, eine Heteroaryl-$C_1$-$C_6$-alkylgruppe, eine Arylgruppe oder eine Heteroarylgruppe steht, |
| R2, R3 und R4 | jeweils unabhängig voneinander für Wasserstoff, eine $C_1$-$C_6$-Alkylgruppe, Halogen oder eine $C_1$-$C_6$-Acylgruppe steht, mit der Massgabe, dass zumindest einer der Reste R2, R3 und R4 für eine $C_1$-$C_6$-Acylgruppe steht, |

$X^-$ für ein physiologisch verträgliches Anion steht,

(ii) mindestens 5 Gew.-%, bezogen auf das Gesamtgewicht des Mittels, Paraffinöl,
(iii) als Oxidationsmittel mindestens Wasserstoffperoxid
(iv) und mindestens eine farbgebende Verbindung, ausgewählt aus Oxidationsfarbstoffvorprodukten enthält.

Unter keratinischen Fasern oder auch-Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Der kosmetische Träger ist bevorzugt wässrig, alkoholisch oder wässrig-alkoholisch. Zum Zwecke der Haarbleiche sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Ein wässriger Träger enthält im Sinne der Erfindung mindestens 40 Gew.-%, insbesondere mindestens 50 Gew.-% Wasser. Unter wässrigalkoholischen Trägern sind im Sinne der vorliegenden Erfindung wasserhaltige Zusammensetzungen, enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether enthalten. Bevorzugt sind dabei alle wasserlöslichen organischen Lösungsmittel.

Als ersten wesentlichen Inhaltstoff enthalten die erfindungsgemäßen Mittel mindestens ein Acylpyridiniumderivat gemäß Formel (I). Beispiele für die als Substituenten der Verbindungen der Formel (I) genannten werden nachfolgend, aber nicht beschränkend genannt: Beispiele für $C_1$-$C_6$-Alkylreste sind die Gruppen $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$. Beispiele für eine $C_2$-$C_6$-Alkenylgruppe sind eine Prop-2-enylgruppe (Allylgruppe), eine 2-Methyl-prop-2-enylgruppe, eine But-3-enylgruppe, eine But-2-enylgruppe, eine Pent-4-enylgruppe oder eine Pent-3-enylgruppe, wobei die Prop-2-enylgruppe bevorzugt ist. Beispiele für eine $C_2$-$C_6$-Hydroxyalkylgruppe sind $-CH_2CH_2OH$, $-CH_2CH_2CH_2OH$, $-CH_2CH(OH)CH_3$ und $-CH_2CH_2CH_2CH_2OH$, wobei die Gruppe $-CH_2CH_2OH$ bevorzugt ist. Beispiele für $C_1$-$C_6$-Alkoxy-$C_2$-$C_6$-alkylgruppen sind die Gruppen $-CH_2CH_2OCH_3$, $-CH_2CH_2CH_2OCH_3$, $-CH_2CH_2OCH_2CH_3$, $-CH_2CH_2CH_2OCH_2CH_3$, $-CH_2CH_2OCH(CH_3)_2$, $-CH_2CH_2CH_2OCH(CH_3)_2$. Beispiele für eine Carboxy-$C_1$-$C_6$-alkylgruppe sind die Carboxymethylgruppe, die 2-Carboxyethylgruppe oder die 3-Carboxypropylgruppe. Beispiele für Aryl-$C_1$-$C_6$-alkylgruppen sind die Benzylgruppe und die 2-Phenylethylgruppe. Beispiele für eine Heteroaryl-$C_1$-$C_6$-alkylgruppe sind die Pyridin-2-ylmethylgruppe, die Pyridin-3-ylmethylgruppe, die Pyridin-4-ylmethylgruppe, die Pyrimidin-2-ylmethylgruppe, die Pyrrol-1-ylmethylgruppe, die Pyrrol-1-ylethylgruppe, die Pyrazol-1-ylmethylgruppe oder die Pyrazol-1-ylethylgruppe. Beispiele für eine Arylgruppe sind die Phenylgruppe, die 1-Naphthylgruppe oder die 2-Naphthylgruppe. Beispiele für eine Heteroarylgruppe sind die Pyridin-2-ylgruppe, die Pyridin-3-ylgruppe, die Pyridin-4-ylgruppe, die Pyrimidin-2-ylgruppe, die Pyrrol-1-ylgruppe, die Pyrrol-2-ylgruppe, die Pyrazol-1-ylgruppe, die Pyrazol-3-ylgruppe oder die Pyrazol-4-ylgruppe. Beispiele einer $C_1$-$C_6$-Acylgruppe sind Acetyl (1-Oxoethyl), 1-Oxo-propyl, 1-Oxo-butyl, 1-Oxo-Pentyl, 1-Oxo-2,2-dimethylpropyl und 1-Oxo-hexyl.

In einer Ausführungsform der vorliegenden Erfindung sind solche Verbindungen gemäß Formel (I) bevorzugt, bei welchen der Rest R1 der allgemeinen Struktur (I) für eine $C_1$-$C_6$-Alkylgruppe, für eine $C_2$-$C_6$-Alkenylgruppe oder für eine $C_2$-$C_6$-Hydroxyalkylgruppe steht. Es ist erfindungsgemäß bevorzugt, wenn der Rest R1 für eine $C_1$-$C_6$-Alkylgruppe, bevorzugt für Methyl, Ethyl, n-Propyl oder Isopropyl und insbesondere bevorzugt für Methyl, steht.

Es hat sich herausgestellt, dass die Acylpyridiniumderivate gemäß Formel (I) erfindungsgemäß besonders vorteilhafte Eigenschaften besitzen, wenn sie die Acyl-Gruppe entweder in 2- oder 4-Position am Pyridin-Ring tragen. Bevorzugte Verbindungen der Formel (I) sind weiterhin solche Verbindungen, bei denen entweder der Rest R2 oder der Rest R4 für eine $C_1$-$C_6$-Acylgruppe, bevorzugt für eine Acetylgruppe, steht. Es ist weiterhin bevorzugt, wenn einer der Reste R2 oder R4 für eine Acetylgruppe steht, während der andere dieser Reste sowie der Rest R3 jeweils für Wasserstoff stehen. Eine weitere Ausführungsform der vorliegenden Erfindung ist daher dadurch gekennzeichnet, dass das Mittel als Acylpyridiniumderivat gemäß Formel (I) mindestens ein 2-Acetylpyridiniumderivat und/oder 4-Acetylpyridiniumderivat enthält. Geeignete Acetylpyridiniumderivate sind dabei insbesondere die physiologisch verträglichen Salze, die als Kation ein Acetylpyridiniumderivat, ausgewählt aus 4-Acetyl-1-methylpyridinium, 4-Acetyl-1-allylpyridinium, 4-Acetyl-1-(2-hydroxyethyl)pyridinium, 2-Acetyl-1-methylpyridinium, 2-Acetyl-1-allylpyridinium und 2-Acetyl-1-(2-hydroxyethyl)pyridinium, enthalten.

Es ist bevorzugt, wenn das Anion $X^-$ gemäß Formel (I) ausgewählt wird aus Halogenid, insbesondere Chlorid, Bromid und Iodid, Benzolsulfonat, p-Toluolsulfonat, $C_1$-$C_4$-Alkylsulfonat, Trifluormethansulfonat, Acetat, Triflluoracetat, Perchlorat, ½ Sulfat, Hydrogensulfat, Tetrafluorborat, Hexafluorphosphat oder Tetrachlorzinkat. Erfindungsgemäß vorteilhaft ist es, wenn das physiologisch verträgliche Anion $X^-$ für ein Halogenidion (insbesondere Chlorid oder Bromid), Hydrogensulfat, p-Toluolsulfonat, Benzolsulfonat oder Acetat steht.

Insbesondere sind solche Mittel erfindungsgemäß bevorzugt, die dadurch gekennzeichnet sind, dass das Acylpyridiniumderivat gemäß Formel (I) ausgewählt ist aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridiniumacetat. Erfindungsgemäß bevorzugte Mittel sind dadurch gekennzeichnet, dass sie als das Acylpyridiniumderivat gemäß Formel (I) eine Verbindung, ausgewählt aus 4-Acetyl-1-methylpyridinium-p-toluolsulfonat und/oder 2-Acetyl-1-methylpyridinium-p-toluolsulfonat, insbesondere 4-Acetyl-1-methylpyridinium-p-toluolsulfonat, enthalten.

Eine Ausführungsform der vorliegenden Erfindung ist dabei dadurch gekennzeichnet, dass im erfindungsgemäßen Mittel die Acylpyridiniumderivate der Formel (I) in einer Menge von 0,1 bis 10 Gew.-%, insbesondere von 0,2 bis 4 Gew.-%, jeweils bezogen das Gesamtgewicht des Mittels, enthalten sind.

Als zweiten wesentlichen Inhaltstoff enthalten die erfindungsgemäßen Mittel weiterhin mindestens 5 Gew.-%, bezogen auf das Gesamtgewicht des Mittels, Paraffinöl.

Es hat sich herausgestellt, dass sich die Farbintensität der Mittel insbesondere dann steigern lässt, wenn das Mittel einen höheren Anteil, wenigstens von 5 Gew.-%, besser von 10 Gew.-%, an Paraffinöl enthält.

Eine Ausführungsform der vorliegenden Erfindung ist daher ein Mittel, welches dadurch gekennzeichnet ist, dass es mindestens 5 Gew.-%, bevorzugt mindestens 10 Gew.-% und besonders bevorzugt von 10 bis 40 Gew.-%, insbesondere von 15 bis 30 Gew.-%, jeweils bezogen auf das Gesamtgewicht des Mittels, Paraffinöl enthält.

Als dritten wesentlichen Bestandteil enthält das erfindungsgemäße Mittel als Oxidationsmittel mindestens Wasserstoffperoxid. Bevorzugt wird Wasserstoffperoxid selbst als wässrige Lösung verwendet. Wasserstoffperoxid kann aber auch in Form einer festen Anlagerungsverbindung von Wasserstoffperoxid an anorganische oder organische Verbindungen, wie Natriumpercarbamid, Polyvinylpyrrolidinon n $H_2O_2$ (n ist eine positive ganze Zahl größer 0), Harnstoffperoxid und Melaminperoxid, eingesetzt werden.

Wasserstoffperoxid ist im anwendungsbereiten Mittel bevorzugt zu 0,1 bis 25 Gew.-%, insbesondere bevorzugt zu 1 bis 20 Gew.-% und besonders bevorzugt zu 4,5 bis 9 Gew.-%, jeweils berechnet auf 100%iges Wasserstoffperoxid und bezogen auf das Gesamtgewicht des anwendungsbereiten Mittels, enthalten.

Als vierten wesentlichen Inhaltstoff enthalten die erfindungsgemäßen Mittel mindestens eine farbgebende Verbindung (iv), ausgewählt aus mindestens einem Oxidationsfarbstoffvorprodukt.

Die erfindungsgemäßen Mittel enthalten als Oxidationsfarbstoffvorprodukt mindestens ein Oxidationsfarbstoffvorprodukt vom Entwicklertyp (Entwicklerkomponente), bevorzugt in Kombination mit mindestens einer Oxidationsfarbstoffvorprodukt vom Kupplertyp (Kupplerkomponente).

Bevorzugte Oxidationsfarbstoffvorprodukte vom Entwicklertyp sind p-Phenylendiaminderivate. Bevorzugte p-Phenylendiamine werden ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Di-hydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1 H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1 H-imidazol-1-yl)propyl]amin, 2-Methoxymethyl-p-phenylendiamin sowie deren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere ausgewählt aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)-ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze, besonders bevorzugt ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder einem ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie Pyrimidinderivaten, Pyrazolderivaten, Pyrazolopyrimidin-und Pyrazolopyrazol-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol.

Bevorzugte Pyrazolopyrimidine sind die Verbindungen, die ausgewählt werden unter Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist. Bevorzugtes-Pyrazolopyrazol-Derivat ist 2,3-Diamino-6,7-dihydro-1H,5H-pyrazolo[1,2-a]pyrazol-1-on.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen. Ganz besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1 H-imidazol-1-yl)propyl]amin, und/oder 4,5-Diamino-1-(2-hydroxyethyl)-pyrazol sowie deren physiologisch verträglichen Salze.

Die Entwicklerkomponenten werden bevorzugt in einer Menge von 0,0001 bis 2,0 Gew.-%, vorzugsweise 0,001 bis 0,5 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäße Kupplerkomponenten werden bevorzugt als mindestens eine Verbindung aus einer der folgenden Klassen ausgewählt: m-Aminophenol, o-Aminophenol, m-Diaminobenzol, o-Diaminobenzol und/oder deren Derivate; Naphthalinderivate mit mindestens einer Hydroxygruppe; Di- beziehungsweise Trihydroxybenzol; Pyridinderivate; Pyrimidinderivate; bestimmte IndolDerivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin);

Chinoxalinderivate (beispielsweise 6-Methyl-1,2,3,4-tetrahydrochinoxalin), sowie Gemische aus zwei oder mehreren Verbindungen aus einer oder mehreren dieser Klassen.

Bevorzugte m-Aminophenol-Kupplerkomponenten werden ausgewählt 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen.

Bevorzugte m-Diaminobenzol-Kupplerkomponenten werden ausgewählt aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol und deren physiologisch verträglichen Salzen.

Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen. Bevorzugte Naphthalinderivate mit mindestens einer Hydroxygruppe werden ausgewählt aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Bevorzugte Di- beziehungsweise Trihydroxybenzole und deren Derivate werden ausgewählt aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Bevorzugte Pyridinderivate werden ausgewählt aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen.

Bevorzugte Pyrimidinderivate werden ausgewählt aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Bevorzugte Indolderivate werden ausgewählt aus 4-Hydroxyindol, 6-Hydroxyindol und 7-Hydroxyindol und deren physiologisch verträglichen Salzen. Bevorzugte Indolinderivate werden ausgewählt aus 4-Hydroxyindolin, 6-Hydroxyindolin und 7-Hydroxyindolin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 1 Gew.-%, vorzugsweise 0,001 bis 0,2 Gew.-%, jeweils bezogen auf das anwendungsbereite Mittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe

benötigen. Direktziehende Farbstoffe können in anionische, kationische und nichtionische direktziehende Farbstoffe unterteilt werden. Üblicherweise sind es Nitrophenylendiamine, Nitroaminophenole, Azofarbstoffe, Anthrachinone oder Indophenole. Die direktziehenden Farbstoffe werden jeweils bevorzugt in einer Menge von 0,0001 bis 1,0 Gew.-%, bevorzugt von 0,001 bis 0,5 Gew.-%, jeweils-bezogen auf die gesamte Anwendungszubereitung, eingesetzt. Die Gesamtmenge an direktziehenden Farbstoffen beträgt vorzugsweise höchstens 0,2 Gew.-%.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

In einer weiteren Ausführungsform der vorliegenden Anmeldung enthalten die erfindungsgemäßen Mittel mindestens einen kationischen, direktziehenden Farbstoff.

Als kationische direktziehende Farbstoffe eignen sich insbesondere 9-(Dimethylamino)benzo[a]-phenoxazin-7-ium-chlorid (C.I. 51,175; Basic Blue 6), Di-[4-(diethylamino)phenyl][4-(ethylamino)-naphthyl]carbenium-chlorid (C.I. 42,595; Basic Blue 7), Di-(4-(dimethylamino)phenyl)-(4-(methylphenylamino)naphthalin-1-yl)carbenium-chlorid (C.I. 42,563; Basic Blue 8), 3,7-Di(dimethylamino)-phenothiazin-5-ium-chlorid (C.I. 52,015 Basic Blue 9), Di-[4-(dimethylamino)phenyl][4-(phenylamino)naphthyl] carbenium-chlorid (C.I. 44,045; Basic Blue 26), 2-[(4-(Ethyl(2-hydroxyethyl)-amino)phenyl)azo]-6-methoxy-3-methyl-benzothiazolium-methylsulfat (C.I. 11,154; Basic Blue 41), 8-Amino-2-brom-5-hydroxy-4-imino-6-[(3-(trimethylammonio)phenyl)amino]-1(4H)-naphthalinon-chlorid (C.I. 56,059; Basic Blue No. 99), Bis[4-(dimethylamino)phenyl]-[4-(methylamino)-phenyl]carbenium-chlorid (C.I. 42,535; Basic Violet 1), Tri(4-amino-3-methylphenyl)carbenium-chlorid (C.I. 42,520; Basic Violet 2), Tri[4-(dimethylamino)-phenyl]carbenium-chlorid (C.I. 42,555; Basic Violet 3), 2-[3,6-(Diethylamino)dibenzopyranium-9-yl]-benzoesäurechlorid (C.I. 45,170; Basic Violet 10), Di(4-aminophenyl)(4-amino-3-methylphenyl)carbeniumchlorid (C.I. 42,510 Basic Violet 14), 1,3-Bis[(2,4-diamino-5-methylphenyl)azo]-3-methylbenzol (C.I. 21,010; Basic Brown 4), 1-[(4-Aminophenyl)azo]-7-(trimethylammonio)- 2-naphthol-chlorid (C.I. 12,250; Basic Brown 16), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (C.I. 12,251; Basic Brown 17), 3-[(4-Amino-2,5-dimethoxyphenyl)azo]-N,N,N-trimethylbenzolaminiumchlorid (C.I. 12,605, Basic Orange 69), 3,7-Diamino-2,8-dimethyl-5-phenylphenazinium-chlorid (C.I. 50,240; Basic Red 2), 1,4-Dimethyl-5-[(4-(dimethylamino)phenyl)azo]-1,2,4-triazolium-chlorid (C.I. 11,055; Basic Red 22), 2-Hydroxy-1-[(2-methoxyphenyl)azo]-7-(trimethylammonio)-naphthalin-chlorid (C.I. 12,245; Basic Red 76), Di[4-(dimethylamino)phenyl]iminomethan-hydrochlorid (C.I. 41,000; Basic Yellow 2), 2-[2-((2,4-Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]-pyrazol-5-on-chlorid (C.I. 12,719; Basic Yellow 57), Bis[4-(diethylamino)phenyl]phenylcarbenium-hydrogensulfat (1:1) (C.I. 42,040; Basic Green 1), Di(4-(dimethylamino)phenyl)phenylmethanol (C.I. 42,000; Basic Green 4), 1-(2-Morpholiniumpropylamino)-4-hydroxy-9,10-anthrachinon-methylsulfat, 1-[(3-(Dimethylpropylaminium)propyl)amino]-4-(methylamino)-9,10-anthrachinon-chlorid und direktziehende Farbstoffe, die einen Heterocyclus enthalten, der mindestens ein quaternäres Stickstoffatom aufweist.

Bevorzugte kationische direktziehende Farbstoffe sind dabei kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, wie in der EP-A2-998 908. Die Verbindungen, die auch unter den Bezeichnungen Basic Yellow 87, Basic Orange 31 und Basic Red 51 bekannt sind, sind ganz besonders bevorzugte kationische direktziehende Farbstoffe. Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Erfindungsgemäß besonders bevorzugte Mittel enthalten als zusätzliche farbgebende Verbindung mindestens einen kationischen Farbstoff, der ausgewählt ist aus Basic Blue 7, Basic Blue 26, Basic Violet 2, Basic Violet 14, Basic Yellow 57, Basic Red 76, Basic Blue 99, Basic Brown 16 und Basic Brown 17 sowie Basic Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Unter Berücksichtigung der bisher genannten bevorzugten Ausführungsformen stellt es eine ganz spezielle und ausdrücklich bevorzugte Ausführungsform dar, wenn das Mittel zum Färben von keratinischen Fasern in einem kosmetischen Träger als erste Komponente mindestens eine Verbindung, ausgewählt aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allyl-pyridinium-benzolsulfonat, 4-Acetyl-1-allylpyridiniumhydrogensulfat, 4-Acetyl-1-allylpyridinium-acetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridiniumacetat, enthält, als zweite Komponente mindestens 10 Gew.-% Paraffinöl (Paraffinum liquidum) enthält, als dritte Komponente Wasserstoffperoxid enthält und als vierte Komponente mindestens ein Oxidationsfarbstoffvorprodukt enthält.

Besonders bevorzugt sind schließlich Mittel, die 0,2 bis 4,0 Gew.-% 4-Acetyl-1-methylpyridinium-p-toluolsulfonat, 10 bis 40 Gew.-% Paraffinöl (Paraffinum liquidum), 2,0 bis 12,0 Gew.-% Wasserstoffperoxid und 0,1 bis 5 Gew.-% einer farbgebenden Verbindung, ausgewählt aus mindestens einem Oxidationsfarbstoffvorprodukt enthalten,
oder Mittel, die 0,2 bis 4,0 Gew.-% 2-Acetyl-1-methylpyridinium-p-toluolsulfonat, 10 bis 40 Gew.-% Paraffinöl (Paraffinum liquidum), 2,0 bis 12,0 Gew.-% Wasserstoffperoxid und 0,1 bis 5 Gew.-% einer farbgebenden Verbindung, ausgewählt aus mindestens einem Oxidationsfarbstoffvorprodukt enthalten,
worin sich die Gewichtsangaben jeweils auf das Gesamtgewicht des Mittels beziehen.

Oxidative Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Bei den pH-Werten im Sinne der vorliegenden Erfindung handelt es sich um pH-Werte, die bei einer Temperatur von 22 °C gemessen wurden.

Andererseits kann es aus Stabilitätsgründen sinnvoll sein, bestimmte Inhaltstoffe bei einem neutralen oder schwach sauren pH-Wert zu lagern und erst zum Zeitpunkt der Anwendung einem alkalischen Milieu auszusetzen. Zu diesen Inhaltstoffen gehört Wasserstoffperoxid, aber auch die kationischen Acylpyridiniumderivate der Formel (I) werden bevorzugt bei neutralen oder schwach sauren pH-Wert gelagert.

Um die Färbeleistung zu verbessern, besitzen die anwendungsbereiten Färbemittel daher bevorzugt einen alkalischen pH-Wert. Daher ist es bevorzugt, ein Mittel des ersten Erfindungsgegenstands unmittelbar vor der Anwendung auf der keratinischen Faser auf einen alkalischen pH-Wert zwischen 6 und 12, insbesondere zwischen 9 und 11 und besonders bevorzugt zwischen 9,5 und 10,5 einzustellen.

Es kann daher bevorzugt sein, wenn das-anwendungsbereite, alkalische Mittel zum Färben der keratinischen Fasern erst unmittelbar vor der Anwendung aus zwei oder mehr Komponenten zusammengemischt wird.

Ein weiterer Gegenstand der vorliegenden Erfindung ist daher ein Verfahren zur Herstellung eines anwendungsbereiten Mittels zum Färben von keratinischen Fasern, insbesondere menschlichen Haaren, welches dadurch gekennzeichnet ist, dass unmittelbar vor Anwendung ein Mittel M1, enthaltend in einem kosmetisch verträglichen Träger

(i) mindestens ein kationisches Acylpyridiniumderivat der Formel (I) gemäß dem ersten Erfindungsgegenstand,
(ii) mindestens 5 Gew.-%, bezogen auf das Gesamtgewicht des Mittels, Paraffinöl,
(iii) und als Oxidationsmittel mindestens Wasserstoffperoxid, und ein Mittel M2, enthaltend in einem kosmetisch verträglichen Träger
(iv) mindestens eine farbgebende Verbindung, ausgewählt aus Oxidationsfarbstoffvorprodukten, und
(v) mindestens ein Alkalisierungsmittel,

miteinander vermischt werden und dass das anwendungsbereite Mittel einen pH-Wert von pH 6 bis pH 12, bevorzugt pH 9,5 bis pH 10,5 besitzt.

Aus Stabilitätsgründen kann es bevorzugt sein, wenn das erfindungsgemäße Mittel einen schwach sauren pH-Wert besitzt. Eine weitere Ausführungsform der vorliegenden Erfindung ist daher dadurch gekennzeichnet, dass das Mittel M1 einen pH-Wert von pH 2 bis pH 6, bevorzugt vom pH 2,5 bis pH 4,5 besitzt.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus Ammoniak, anorganischen Salzen, insbesondere der Alkali- und Erdalkalimetalle, organischen Alkalisierungsmitteln, insbesondere Alkanolaminen, Aminen und basische Aminosäuren. Erfindungsgemäß bevorzugte Acidifizierungsmittel sind Genuss-Säuren, wie Milchsäure, Zitronensäure, Essigsäure, Äpfelsäure oder Weinsäure, verdünnte Mineralsäuren und deren in Wasser sauer reagierenden Salze sowie organische Phosphon- oder Sulfonsäuren.

Erfindungsgemäß kann das Mittel zusammen mit einem Katalysator auf das Haar aufgebracht werden, der die Oxidation der Farbstoffvorprodukte zusätzlich aktiviert. Solche Katalysatoren sind z. B. bestimmte Enzyme, Iodide, Chinone oder Metallionen. Hierfür geeignete Enzyme sind z. B. Peroxidasen, die die Wirkung geringer Mengen an Wasserstoffperoxid deutlich verstärken können. Ein Einsatz bestimmter Metallionen oder -komplexe kann ebenfalls

bevorzugt sein. Besonders geeignet sind dabei $Zn^{2+}$, $Cu^{2+}$ und $Mn^{2+}$.

Weiterhin hat es sich als vorteilhaft erweisen, wenn die Mittel mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-atomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/ oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die erfindungsgemäß verwendbaren Alkalisierungsmittel (v) des Mittels M2 werden bevorzugt ausgewählt aus der Gruppe, die gebildet wird aus Ammoniak, anorganischen Alkalisierungsmitteln, insbesondere der Alkali- und Erdalkalimetalle, organischen Alkalisierungsmitteln, insbesondere Alkanolaminen, Aminen und basische Aminosäuren.

Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Besonders bevorzugt sind Natriumhydroxid und/oder Kaliumhydroxid. D

Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin.

Besonders bevorzugt als Alkalisierungsmittel enthält das Mittel M2 jedoch Ammoniak. Eine bevorzugte Ausführungsform der vorliegenden Erfindung ist daher dadurch gekennzeichnet, dass das Mittel M2 als Alkalisierungsmittel Ammoniak enthält.

Erfindungsgemäße, anwendungsbereite Mittel sind vorzugsweise wässrige, fließfähige Zubereitungen. Die erfindungsgemäßen Mittel können weiterhin alle für solche Zubereitungen bekannten Wirk-, Zusatz- und Hilfsstoffe enthalten. Die anwendungsbereiten Mittel als Mischung aus Mittel M1 und M2 können dabei oberflächenaktive Substanzen, ausgewählt aus anionischen sowie nichtionischen, zwitterionischen, amphoteren und kationischen Tensiden enthalten.

Anionische Tenside sind gekennzeichnet durch eine wasserlöslich machende, anionische Gruppe wie eine Carboxylat-, Sulfat-, Sulfonat- oder Phosphat-Gruppe und eine lipophile Alkylgruppe mit etwa 8 bis 30 C-Atomen. Beispiele lineare und verzweigte Fettsäuren mit 8 bis 30 C-Atomen (Seifen); Ethercarbonsäuren; Acylsarcoside; Acyltauride; Acylisethionate; Sulfobernsteinsäurealkylester; lineare Alkansulfonate; lineare $\alpha$-Olefinsulfonate; Sulfonate ungesättigter Fettsäuren; $\alpha$-Sulfofettsäuremethylester von Fettsäuren; Alkylsulfate und Alkylethersulfate, insbesondere der Formel $RO(CH_2CH_2O)_xSO_3H$, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate; sulfatierte Fettsäurealkylenglykolester sowie Monoglyceridsulfate und Monoglyceridethersulfate.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Beispiele solcher zwitterionischen Tenside sind die Betaine wie die N-Alkyl-N,N-dimethylammonium-glycinate, N-Acylaminopropyl-N,N-dimethylammoniumglycinate, und 2-Alkyl-3-carboxymethyl-3-hydroxyethyl-imidazoline mit jeweils 8 bis 18 C-Atomen in der Alkyl- oder Acylgruppe sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Übliche amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Beispielhafte amphotere Tenside sind N-Kokosalkylaminopropionat, Kokosacylaminoethylaminopropionat und $C_{12}$-$C_{18}$-Acylsarcosin.

Nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 1 bis 50 Mol Ethylenoxid und/oder 0 bis 5 Mol Propylenoxid an lineare und verzweigte Fettalkohole, an Fettsäuren und an Alkylphenole; mit einem Methyl- oder $C_2$-$C_6$-Alkylrest endgruppenverschlossene Anlagerungsprodukte von 1 bis 50 Mol Ethylenoxid und/oder 0 bis 5 Mol Propylenoxid an lineare und verzweigte Fettalkohole, an Fettsäuren und an Alkylphenole; Polyglycerinester und alkoxylierte Polyglycerinester; Polyolfettsäureester; höher alkoxylierte, propoxylierte und ethoxylierte, Mono-, Di- und Triglyceride, wie beispielsweise Glycerinmonolaurat + 20 Ethylenoxid und Glycerinmonostearat + 20 Ethylenoxid; Aminoxide; Hydroxymischether; Sorbitanfettsäureester wie Polysorbate und Sorbitanmonolaurat + 20 Mol Ethylenoxid (EO); Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate; Alkylpolyglykoside entsprechend der allgemeinen Formel RO-$(Z)_x$, wobei R für Alkyl, Z für Zucker sowie x für die Anzahl der Zuckereinheiten steht.

Erfindungsgemäß bevorzugt sind in anwendungsbereiten Mitteln kationische Tenside vom Typ der quartären Ammoniumverbindungen, der Esterquats und der Amidoamine, wie Stearamidopropyldimethylamin. Bevorzugte quaternäre Ammoniumverbindungen sind Ammoniumhalogenide, wie Alkyltrimethylammoniumchloride, Dialkyldimethylammoniumchloride und Trialkylmethylammoniumchloride sowie die unter den INCI-Bezeichnungen Quaternium-27 und Quaternium-83 bekannten Imidazolium-Verbindungen. Weitere erfindungsgemäße kationische Tenside stellen die quaternisierten Proteinhydrolysate dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen, wie die Produkte Armocare VGH-70, ein N,N-Bis(2-Palmitoyloxyethyl)dimethylammoniumchlorid, sowie Dehyquart F-75, DehyquartC-4046, Dehyquart L80 und Dehyquart AU-35.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können anionische, nicht-ionische, zwitterionische und/oder amphotere Tenside sowie deren Mischungen bevorzugt sein.

Die Mittel M1 und/oder M2 können weitere Wirk-, Hilfs- und Zusatzstoffe enthalten. Weitere, erfindungsgemäß einsetzbare Wirk-, Hilfs- und Zusatzstoffe sind beispielsweise anionische Polymere (wie Carbomere, Co- und Crosspolymere von Acrylsäure, Methacrylsäure, Maleinsäure, Itaconsäure und gegebenenfalls weiteren nichtionischen Monomeren); nichtionische Polymere (wie Vinylpyrrolidinon/Vinylacrylat-Copolymere, Polyvinylpyrrolidinon und Vinylpyrrolidinon/VinylacetatCopolymere und Polysiloxane); zwitterionische und amphotere Polymere (wie Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere); Verdickungsmittel (wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie Bentonit oder vollsynthetische Hydrokolloide wie z.B. Polyvinylalkohol); Strukturanden (wie Zucker, Maleinsäure und Milchsäure) und Konsistenzgeber (wie Zuckerester, Polyolester oder Polyolalkylether); Proteinhydrolysate (insbesondere Elastin-, Kollagen-, Keratin-, Milcheiweiß-, Sojaprotein- und Weizenproteinhydrolysate, deren Kondensationsprodukte mit Fettsäuren); Parfümöle; Pflegeöle; Cyclodextrine; Entschäumer wie Silicone; Farbstoffe und Pigmente zum Anfärben des Mittels; Antischuppenwirkstoffe (wie Piroctone Olamine, Zink Omadine und Climbazol); Lichtschutzmittel (insbesondere derivatisierte Benzophenone, Zimtsäure-Derivate und Triazine); Wirkstoffe (wie Allantoin, Pyrrolidoncarbonsäuren, Cholesterin und deren Salze); weitere Fette und Wachse (wie Fettalkohole, Bienenwachs, Montanwachs und Paraffine); Quell- und Penetrationsstoffe (wie Glycerin, Propylenglykolmonoethylether, Carbonate, Hydrogencarbonate, Guanidine, Harnstoffe sowie primäre, sekundäre und tertiäre Phosphate); Trübungsmittel (wie Latex, Styrol/PVP- und Styrol/Acrylamid-Copolymere); Perlglanzmittel (wie Ethylenglykolmono- und -distearat sowie PEG-3-distearat); Treibmittel (wie Propan-Butan-Gemische, $N_2O$, Dimethylether, $CO_2$ und Luft) sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die bevorzugten Ausführungsformen des ersten Erfindungsgegenstands sind mutatis mutandis auf den zweiten Erfindungsgegenstand anwendbar.

Das erfindungsgemäße Mittel wird in einer Ausführungsform der Erfindung durch Vermischen der Alkalisierungszubereitung M2 und der Oxidationszubereitung M1 unmittelbar vor der Anwendung hergestellt. Es ist daher vorteilhaft, dem Anwender beide Zubereitungen in einem Set anzubieten.

Daher ist eine bevorzugte Darreichungsform des anwendungsbereiten Mittels eine getrennte Verpackungseinheit, worin die Mittel M1 und M2 jeweils getrennt voneinander verpackt vorliegen.

Ein weiterer Gegenstand der vorliegenden Erfindung ist daher eine Mehrkomponentenverpackungseinheit (Kit-of-Parts), umfassend mindestens zwei getrennt voneinander konfektionierte Container, wobei ein erster Container C1 mindestens ein Mittel M1, enthaltend in einem kosmetischen Träger

(i) mindestens ein kationisches Acylpyridiniumderivat der Formel (I) gemäß dem ersten Erfindungsgegenstand,
(ii) mindestens 5 Gew.-%, bezogen auf das Gesamtgewicht des Mittels, Paraffinöl,
(iii) und als Oxidationsmittel mindestens Wasserstoffperoxid,

und ein zweiter Container C2 mindestens ein Mittel M2, enthaltend in einem kosmetisch verträglichen Träger

(iv) mindestens eine farbgebende Verbindung, ausgewählt aus Oxidationsfarbstoffvorprodukten, und
(v) mindestens ein Alkalisierungsmittel,

beinhaltet.

Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff.

Weiterhin kann es erfindungsgemäß besonders vorteilhaft sein, wenn das genannte Kit-of-Parts mindestens ein weiteres Haarbehandlungsmittel in einem getrennten Container enthält, insbesondere eine Konditioniermittelzubereitung oder ein Blondierpulver mit Peroxodisulfaten. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Bevorzugte Mehrkomponentenverpackungseinheiten sind dadurch gekennzeichnet, dass das Mittel M1 in Container C1 einen pH-Wert von pH 2 bis pH 6, insbesondere von pH 2,5 bis pH 4,5 besitzt.

Weiterhin bevorzugte Mehrkomponentenverpackungseinheiten sind dadurch gekennzeichnet, dass das Mittel M2 in Container C2 als Alkalisierungsmittel mindestens Ammoniak enthält.

Hinsichtlich der bevorzugten Ausführungsführungsformen der Mittel M1 und M2 gelten mutatis mutandis die obigen Ausführungen der vorangehenden Erfindungsgegenstände.

Ein weiterer Erfindungsgegenstand ist ein Verfahren zur oxidativen Färbung keratinischer Fasern, insbesondere menschlicher Haare, welches dadurch gekennzeichnet ist, dass aus einer Mehrkomponentenverpackungseinheit gemäß dem vorangegangenen Erfindungsgegenstand die beiden Mittel M1 und M2 in einem der Container C2 oder C1 vereinigt werden, der wiederverschlossene Container daraufhin geschüttelt wird, und das im Container resultierende, anwendungsbereite Farbveränderungsmittel anschließend auf die Fasern aufgebracht wird, für eine Einwirkdauer von 5 bis 60 min, bevorzugt 10 bis 35 min auf den Fasern belassen und schließlich ausgespült wird.

Die Anwendungstemperaturen können in einem Bereich zwischen 15 und 40 °C liegen. Nach der Einwirkungszeit wird das verbleibende Mittel durch Ausspülen von dem Haar entfernt. Das Nachwaschen mit einem Shampoo entfällt, wenn ein stark tensidhaltiger Träger verwendet wurde.

Im Rahmen dieses Gegenstandes der Erfindung gelten mutatis mutandis die oben getroffenen Aussagen analog.

Ein weiterer Gegenstand der vorliegenden Erfindung ist die kosmetische Verwendung einer Kombination aus mindestens einem kationischens Acylpyridiniumderivat der Formel (I) gemäß Anspruch 1 und mindestens 5 Gew.-%, bezogen auf das Gesamtgewicht des Mittels, Paraffinöl in Färbemitteln für keratinhaltige Fasern, insbesondere menschliche Haare, zur Farbintensivierung bei der oxidativen Färbung.

Beispiele

1.1 Herstellung von 2 Färbecremes (Tabelle 1)

| Rohstoff | Färbecreme 1 [Gew.-%] | Färbecreme 2 [Gew.-%] |
|---|---|---|
| 4,5-Diamino-1-(2-hydroxyethyl)pyrazolsulfat | 1,08 | 0,72 |

(fortgesetzt)

| Rohstoff | Färbecreme 1 [Gew.-%] | Färbecreme 2 [Gew.-%] |
|---|---|---|
| 4-Amino-3-methylphenol | 0,11 | -- |
| 5-Amino-2-methylphenol | 0,19 | 0,34 |
| 3-Aminophenol | 0,42 | |
| 2,7-Dihydroxynaphthalin | -- | 0,04 |
| Basic Red 51 | 0,50 | -- |
| Basic Orange 31 | -- | 0,50 |
| Ammoniumcarbomer, wässrig, 1,0 Gew.-% | 15,00 | 15,00 |
| Natrium Cetearylsulfat | 0,70 | 0,70 |
| Natrium Laurylethersulfat (2 EO), wässriq, Gew.-27% | 4,40 | 4,40 |
| Kalium Oleat, wässrig, 12,5 Gew.-% | 3,00 | 3,00 |
| Cetearyl Alcohol | 12,00 | 12,00 |
| Ceteareth-20 | 3,00 | 3,00 |
| 2-Octyldodecanol | 2,00 | 2,00 |
| Cutina AGS | 2,00 | 2,00 |
| Cutina GMS SE | 2,00 | 2,00 |
| Titandioxid | 0,50 | 0,50 |
| Phospholipid EFA | 0,10 | 0,10 |
| Tetranatrium EDTA | 0,20 | 0,20 |
| Merquat Plus 3330 | 0,50 | 0,50 |
| Ascorbinsäure | 0,05 | 0,05 |
| Puricare LS 9658 | 1,00 | 1,00 |
| Monoethanolamin | 1,00 | 1,00 |
| Parfum | qs | qs |
| Ammoniak, 25 Gew.-%, wässrig | 15,00 | 15,00 |
| Wasser | ad 100 | ad 100 |
| *Rohstoffe: Cutina AGS (INCI-Bezeichnung: Glycol Distearate (Cognis)); Cutina GMS SE (INCI-Bezeichnung: Glyceryl Stearate (Cognis)); Phospholipid EFA (ca. 30%, INCI-Bezeichnung: Linoleamidopropyl PG-dimonium chloride phosphate, Propylene glycol (Uniqema)); Merquat Plus 3330 (ca. 10% ; INCI-Bezeichnung: Polyquaternium-39 (Nalco)); Puricare LS 9658 (ca. 1%; INCI-Bezeichnung: Glycerin, Moringa Pterygosperma Seed Extrakt (Laboratoires Serobiologiques). | | |

1.2 Oxidationsmittelzubereitungen (Tabelle 2)

| Rohstoff | Oxidationsmittelzubereitung [Gew.-%] | | | |
|---|---|---|---|---|
| | E1 (Vgl.) | E2 (Vgl.) | E3 (Vgl.) | E4 (Erf.) |
| Natriumbenzoat | 0,04 | 0,04 | 0,04 | 0,04 |
| Dinatriumpyrophosphat | 0,10 | 0,10 | 0,10 | 0,10 |
| Etidronsäure, wässriq, 60 Gew.-% | 0,25 | 0,25 | 0,25 | 0,25 |
| Kaliumhydroxid, wässrig, 50 Gew.-% | 0,12 | 0,12 | 0,12 | 0,12 |
| Cetearylalkohol | 4,00 | 4,00 | 4,00 | 4,00 |

(fortgesetzt)

| **Rohstoff** | **Oxidationsmittelzubereitung [Gew.-%]** | | | |
|---|---|---|---|---|
| | **E1 (Vgl.)** | **E2 (Vgl.)** | **E3 (Vgl.)** | **E4 (Erf.)** |
| Ceteareth-20 | 1,00 | 1,00 | 1,00 | 1,00 |
| Bienenwachs | 0,30 | 0,30 | 0,30 | 0,30 |
| Paraffinum Liquidum | -- | 20,00 | -- | 20,00 |
| 4-Acetyl-1-methylpyridinium-p-toluolsulfonat | -- | -- | 2,00 | 2,00 |
| Wasserstoffperoxid, wässrig, 50 Gew.-% | 23,30 | 23,30 | 23,30 | 23,30 |
| Wasser | ad 100 | ad 100 | ad 100 | ad 100 |

Bei den Rezepturen E1 bis E3 handelt es sich um nicht erfindungsgemäße Vergleichsrezepturen, die Rezepturen E4 ist ein erfindungsgemäßes Beispiel mit 4-Acetyl-1-methylpyridinium-p-toluolsulfonat und Paraffinum Liquidum. Die anwendungsbereiten Mittel wurden durch Vermischen jeweils der Färbecreme mit einer Oxidationsmittelzubereitung im Verhältnis von 1 zu 2 Gewichtsteilen hergestellt. Diese Mittel besitzen einen pH-Wert zwischen 9,5 bis 10,5.

Für den Färbeprozess wurde auf Strähnen dunkelblonden und braunen Haares (Codes: Kerling NH 6/0 bzw. Kerling NH 3/0) von ca. 0,7 g Gewicht die 4-fache Menge der fertigen Anwendungsmischung appliziert. Nachdem die Strähnen für 30 Minuten bei 32 °C gefärbt wurden, wurden sie mit einem handelsüblichen Shampoo gewaschen und mit einem Föhn getrocknet.

2.1 Auswertung der Farbintensivierung

Jede Haarsträhne wurde vor und nach dem Färbevorgang farbmetrisch vermessen. Die farbmetrischen Messungen erfolgten auf der Strähne an jeweils 4 Messpunkten. Als Messgerät diente der Spectralflash SF 450 der Firma Datacolor.

Die Ergebnisse der Messungen wurden mithilfe des CIELAB Farbenraums quantifiziert.
Der a-Wert steht für die rot-grün-Achse des Systems; je größer der Wert ist, umso mehr ist die Färbung ins Rote verschoben. Der b-Wert steht für die gelb-blau-Achse des Systems; je größer der Wert ist, umso mehr ist die Färbung ins Gelbe verschoben.

Als Maß für die Farbintensivierung der jeweiligen Rezeptur wurde der Abstand zwischen der Farbigkeit (Chroma) der unbehandelten Strähne und der Farbigkeit der gefärbten Strähne herangezogen.

Das Chroma C kann nach der Formel $C = \sqrt{a^2 + b^2}$ berechnet werden.

Je größer der ΔC-Wert ist, der den Unterschied in Farbigkeit von unbehandelten zu gefärbten Fasern angibt, desto besser ist die Farbintensivierung der Färbung durch die jeweilige Rezeptur.

Farbintensivierung bei braunen Strähnen (Kerling 3/0)

| Mittel | Oxidationsmittel M1 | Färbecreme M2 | a-Wert | b-Wert | C-Wert | Farbintensivierung [ΔC] |
|---|---|---|---|---|---|---|
| **3-0** | unbehandelt | | 1,36 | 0,96 | 1,7 | -- |
| **3-1a** | E1 | FC1 | 11,35 | 4,93 | **12,4** | **10,7** |
| **3-1b** | E2 | FC1 | 11,15 | 3,25 | **11,6** | **9,9** |
| **3-1c** | E3 | FC1 | 9,36 | 2,48 | **9,7** | **8,0** |
| **3-1d** | E4 | FC1 | 13,30 | 5,24 | **14,3** | **12,6** |
| **3-2a** | E1 | FC2 | 12,50 | 5,29 | **13,6** | **11,9** |
| **3-2b** | E2 | FC2 | 15,34 | 7,14 | **16,9** | **15,2** |
| **3-2c** | E3 | FC2 | 15,56 | 7,53 | **17,3** | **15,6** |
| **3-2d** | E4 | FC2 | 19,45 | 9,65 | **21,7** | **20,0** |

Farbintensivierung bei dunkelblonden Strähnen (Kerling 6/0)

| Mittel | Oxidationsmittel M1 | Färbecreme M2 | a-Wert | b-Wert | C-Wert | Farbintensivierung [ΔC] |
|---|---|---|---|---|---|---|
| **6-0** | unbehandelt | | 5,41 | 8,56 | 10,1 | -- |
| **6-1a** | E1 | FC1 | 21,05 | 7,91 | **22,5** | **12,4** |
| **6-1b** | E2 | FC1 | 22,63 | 8,44 | **24,1** | **14,0** |
| **6-1c** | E3 | FC1 | 19,40 | 6,70 | **20,5** | **10,4** |
| **6-1d** | E4 | FC1 | 23,38 | 9,75 | **25,3** | **15,2** |
| **6-2a** | E1 | FC2 | 26,49 | 13,59 | **29,7** | **19,6** |
| **6-2b** | E2 | FC2 | 30,24 | 17,21 | **34,8** | **24,7** |
| **6-2c** | E3 | FC2 | 29,30 | 18,15 | **34,4** | **24,3** |
| **6-2d** | E4 | FC2 | 31,87 | 18,70 | **36,9** | **26,8** |

2.2 Deutung der Ergebnisse

Eine Abschätzung der Färbeleistungen der unterschiedlichen Rezepturen lässt sich durch den Vergleich der ΔC-Werte treffen. Es ist eindeutig ersichtlich, dass mit der erfindungsgemäßen Kombination aus Paraffinöl und Acylpyridiniumderivat auf unterschiedlichen Haartypen und mit unterschiedlichen Farbstoff-Kombinationen jeweils signifikant größere ΔC Werte - und damit eine verbesserte Farbintensität - erzielt werden konnten, als es ohne Zusatz oder durch den Einsatz von einer der beiden Komponenten möglich war.

## Patentansprüche

1. Mittel zum Färben von keratinischen Fasern, **dadurch gekennzeichnet, dass** es in einem kosmetischen Träger

   (i) mindestens ein kationisches Acylpyridiniumderivat der Formel (I),

   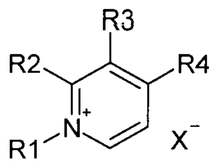
   (I),

   worin

   R1 für eine $C_1$-$C_6$-Alkylgruppe, eine $C_2$-$C_6$-Alkenylgruppe, eine $C_2$-$C_6$-Hydroxy-alkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe steht,
   R2, R3 und R4 jeweils unabhängig voneinander für Wasserstoff, eine $C_1$-$C_6$-Alkylgruppe, ein Halogenatom oder eine $C_1$-$C_6$-Acylgruppe steht, mit der Massgabe, dass zumindest einer der Reste R2, R3 und R4 für eine $C_1$-$C_6$-Acylgruppe steht,
   $X^-$ für ein physiologisch verträgliches Anion steht,

   (ii) mindestens 5 Gew.-%, bezogen auf das Gesamtgewicht des Mittels, Paraffinöl,
   (iii) als Oxidationsmittel mindestens Wasserstoffperoxid
   (iv) und mindestens eine farbgebende Verbindung, ausgewählt aus Oxidationsfarbstoffvorprodukten enthält.

2. Mittel nach Anspruch 1, **dadurch gekennzeichnet, dass** mindestens eine Verbindung der Formel (I) enthalten ist, die ausgewählt wird aus 4-Acetyl-1-methylpyridinium-p-toluolsulfonat und 2-Acetyl-1-methylpyridinium-p-toluolsulfonat.

3. Mittel nach einem der Ansprüche 1 bis 2, **dadurch gekennzeichnet, dass** die Acylpyridiniumderivate der Formel (I) in einer Menge von 0,1 bis 10 Gew.-%, insbesondere von 0,2 bis 4 Gew.-%, jeweils bezogen das Gesamtgewicht des Mittels, enthalten sind.

4. Mittel nach einem der Ansprüche 1 bis 3, **dadurch gekennzeichnet, dass** es in einem kosmetischen Träger als erste Komponente mindestens eine Verbindung, ausgewählt aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridiniumacetat, als zweite Komponente mindestens 10 Gew.-% Paraffinöl, als dritte Komponente Wasserstoffperoxid und als vierte Komponente mindestens ein Oxidationsfarbstoffvorprodukt enthält.

5. Mittel nach einem der Ansprüche 1 bis 3, **dadurch gekennzeichnet, dass** es von 10 bis 40 Gew.-%, insbesondere von 15 bis 30 Gew.-%, bezogen auf das Gesamtgewicht des Mittels, Paraffinöl enthält.

6. Verfahren zur Herstellung eines anwendungsbereiten Mittels zum Färben von keratinischen Fasern, insbesondere menschlichen Haaren, **dadurch gekennzeichnet, dass** es unmittelbar vor Anwendung eines Mittels M1, enthaltend in einem kosmetisch verträglichen Träger

   (i) mindestens ein kationisches Acylpyridiniumderivat der Formel (I) gemäß Anspruch 1,
   (ii) mindestens 5 Gew.-%, bezogen auf das Gesamtgewicht des Mittels, Paraffinöl,
   (iii) und als Oxidationsmittel mindestens Wasserstoffperoxid, und eines Mittels M2, enthaltend in einem kosmetisch verträglichen Träger
   (iv) mindestens eine farbgebende Verbindung, ausgewählt aus Oxidationsfarbstoffvorprodukten, und
   (v) mindestens ein Alkalisierungsmittel,

   miteinander vermischt werden und dass das anwendungsbereite Mittel einen pH-Wert von pH 6 bis pH 12, bevorzugt pH 9,5 bis pH 10,5 besitzt.

7. Verfahren nach Anspruch 6, **dadurch gekennzeichnet, dass** das Mittel M1 einen pH-Wert von pH 2 bis pH 6 besitzt.

8. Verfahren nach Anspruch 7, **dadurch gekennzeichnet, dass** das Mittel M2 als Alkalisierungsmittel mindestens Ammoniak enthält.

9. Mehrkomponentenverpackungseinheit (Kit-of-Parts), umfassend mindestens zwei getrennt voneinander konfektionierte Container, wobei ein erster Container C1 mindestens ein Mittel M1, enthaltend in einem kosmetisch verträglichen Träger

   (i) mindestens ein kationisches Acylpyridiniumderivat der Formel (I) gemäß Anspruch 1,
   (ii) mindestens 5 Gew.-%, bezogen auf das Gesamtgewicht des Mittels, Paraffinöl,
   (iii) und als Oxidationsmittel mindestens Wasserstoffperoxid, und ein zweiter Container C2 mindestens ein Mittel M2, enthaltend in einem kosmetisch verträglichen Träger
   (iv) mindestens eine farbgebende Verbindung, ausgewählt aus Oxidationsfarbstoffvorprodukten, und
   (v) mindestens ein Alkalisierungsmittel,

   beinhaltet.

10. Mehrkomponentenverpackungseinheit (Kit-of-Parts), umfassend mindestens zwei getrennt voneinander konfektionierte Container, wobei ein erster Container C1 mindestens ein Mittel M1, enthaltend in einem kosmetisch verträglichen Träger

   (i) mindestens ein kationisches Acylpyridiniumderivat der Formel (I) gemäß Anspruch 1,
   (ii) mindestens 5 Gew.-%, bezogen auf das Gesamtgewicht des Mittels, Paraffinöl,
   (iii) und als Oxidationsmittel mindestens Wasserstoffperoxid, wobei das Mittel M1 einen pH-Wert von pH 2 bis pH 6 besitzt, und ein zweiter Container C2 mindestens ein Mittel M2, enthaltend in einem kosmetisch verträglichen Träger

(iv) mindestens eine farbgebende Verbindung, ausgewählt aus Oxidationsfarbstoffvorprodukten, und
(v) mindestens ein Alkalisierungsmittel,

beinhaltet.

**11.** Mehrkomponentenverpackungseinheit (Kit-of-Parts), umfassend mindestens zwei getrennt voneinander konfektionierte Container, wobei ein erster Container C1 mindestens ein Mittel M1, enthaltend in einem kosmetisch verträglichen Träger

(i) mindestens ein kationisches Acylpyridiniumderivat der Formel (I) gemäß Anspruch 1,
(ii) mindestens 5 Gew.-%, bezogen auf das Gesamtgewicht des Mittels, Paraffinöl,
(iii) und als Oxidationsmittel mindestens Wasserstoffperoxid,

und ein zweiter Container C2 mindestens ein Mittel M2, enthaltend in einem kosmetisch verträglichen Träger

(iv) mindestens eine farbgebende Verbindung, ausgewählt aus Oxidationsfarbstoffvorprodukten, und
(v) mindestens ein Alkalisierungsmittel, wobei als Alkalisierungsmittel mindestens Ammoniak enthalten ist,

beinhaltet.

**12.** Kosmetische Verwendung einer Kombination aus mindestens einem kationischen Acylpyridiniumderivat der Formel (I) gemäß Anspruch 1 und mindestens 5 Gew.-%, bezogen auf das Gesamtgewicht des Mittels, Paraffinöl in Färbemitteln für keratinhaltige Fasern, insbesondere menschliche Haare, zur Farbintensivierung bei der oxidativen Färbung.

## Claims

**1.** An agent for coloring keratinic fibers, **characterized in that** a cosmetic carrier contains

(i) at least one cationic acylpyridinium derivative of formula (I),

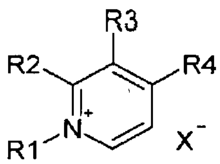

(I),

where R1 is a $C_1$-$C_6$ alkyl group, a $C_2$-$C_6$ alkenyl group, a $C_2$-$C_6$ hydroxyalkyl group, a $C_1$-$C_6$ alkoxy $C_2$-$C_6$ alkyl group, a carboxy $C_2$-$C_6$ alkyl group, an aryl $C_1$-$C_6$ alkyl group, a heteroaryl $C_1$-$C_6$ alkyl group, an aryl group or a heteroaryl group,
R2, R3 and R4 are each independently hydrogen, a $C_1$-$C_6$ alkyl group, a halogen atom or a $C_1$-$C_6$ acyl group, provided that at least one of the functional groups R2, R3 and R4 is a $C_1$-$C_6$ acyl group,
$X^-$ is a physiologically acceptable anion,

(ii) at least 5 wt.%, based on the total weight of the agent, paraffin oil,
(iii) at least hydrogen peroxide as oxidizing agent
(iv) and at least one color-imparting compound, selected from oxidation dye precursors.

**2.** The agent according to claim 1, **characterized in that** at least one compound of formula (I) is contained that is selected from 4-acetyl-1-methylpyridinium-p-toluolsulfonate and 2-acetyl-1-methylpyridinium-p-toluolsulfonate.

**3.** The agent according to one of claims 1 to 2, **characterized in that** the acylpyridinium derivatives of formula (I) are contained in an amount of from 0.1 to 10 wt.%, in particular from 0.2 to 4 wt.%, in each case based on the total weight of the agent.

**4.** The agent according to one of claims 1 to 3, **characterized in that** a cosmetic carrier contains, as a first component, at least one compound, selected from the group comprising 4-acetyl-1-methylpyridinium-p-toluolsulfonate, 4-acetyl-1-methylpyridinium benzenesulfonate, 4-acetyl-1-methylpyridinium hydrogen sulfate, 4-acetyl-1-methylpyridinium acetate, 4-acetyl-1-allylpyridinium-p-toluenesulfonate, 4-acetyl-1-allylpyridinium benzenesulfonate, 4-acetyl-1-allylpyridinium hydrogensulfate, 4-acetyl-1-allylpyridinium acetate, 2-acetyl-1-methylpyridinium-p-toluenesulfonate, 2-acetyl-1-methylpyridinium benzenesulfonate, 2-acetyl-1-methylpyridinium hydrogen sulfate, 2-acetyl-1-methylpyridinium acetate, 2-acetyl-1-allyl-pyridinium-p-toluenesulfonate, 2-acetyl-1-allylpyridinium benzenesulfonate, 2-acetyl-1-allyl-pyridinium hydrogen sulfate and 2-acetyl-1-allylpyridinium acetate, as a second component at least 10 wt.% paraffin oil, as a third component hydrogen peroxide and as a fourth component at least one oxidation dye precursor.

**5.** The agent according to one of claims 1 to 3, **characterized in that** it contains from 10 to 40 wt.%, in particular from 15 to 30 wt.%, based on the total weight of the agent, paraffin oil.

**6.** A method for preparing a ready-to-apply agent for coloring keratinic fibers, in particular human hair, **characterized in that**, immediately before application, an agent M1, containing in a cosmetically acceptable carrier

(i) at least one cationic acylpyridinium derivative of formula (I) according to claim 1,
(ii) at least 5 wt.%, based on the total weight of the agent, paraffin oil
(iii) and at least hydrogen peroxide as oxidizing agent,

and an agent M2, containing in a cosmetically acceptable carrier

(iv) at least one color-imparting compound, selected from oxidation dye precursors, and
(v) at least one alkalizing agent,

are mixed together and **in that** the ready-to-apply agent has a pH of from pH 6 to pH 12, preferably pH 9.5 to pH 10.5.

**7.** The method according to claim 6, **characterized in that** the agent M1 has a pH of from pH 2 to pH 6.

**8.** The method according to claim 7, **characterized in that** the agent M2 contains at least ammonia as alkalizing agent.

**9.** A kit of parts comprising at least two separately assembled containers, wherein a first container C1 contains at least one agent M1, containing in a cosmetically acceptable carrier

(i) at least one cationic acylpyridinium derivative of formula (I) according to claim 1,
(ii) at least 5 wt.%, based on the total weight of the agent, paraffin oil
(iii) and at least hydrogen peroxide as oxidizing agent,

and a second container C2 contains at least one agent M2, containing in a cosmetically acceptable carrier

(iv) at least one color-imparting compound, selected from oxidation dye precursors, and
(v) at least one alkalizing agent.

**10.** The kit of parts, comprising at least two separately assembled containers, wherein a first container C1 contains at least one agent M1, containing in a cosmetically acceptable carrier

(i) at least one cationic acylpyridinium derivative of formula (I) according to claim 1,
(ii) at least 5 wt.%, based on the total weight of the agent, paraffin oil
(iii) and at least hydrogen peroxide as oxidizing agent, wherein the agent M1 has a pH of from pH 2 to pH 6,

and a second container C2 contains at least one agent M2, containing in a cosmetically acceptable carrier

(iv) at least one color-imparting compound, selected from oxidation dye precursors, and
(v) at least one alkalizing agent.

**11.** The kit of parts, comprising at least two separately assembled containers, wherein a first container C1 contains at

least one agent M1, containing in a cosmetically acceptable carrier

(i) at least one cationic acylpyridinium derivative of formula (I) according to claim 1,
(ii) at least 5 wt.%, based on the total weight of the agent, paraffin oil,
(iii) and at least hydrogen peroxide as oxidizing agent,

and a second container C2 contains at least one agent M2, containing in a cosmetically acceptable carrier

(iv) at least one color-imparting compound, selected from oxidation dye-precursors, and
(v) at least one alkalizing agent, wherein at least ammonia is contained as alkalizing agent.

**12.** The cosmetic use of a combination of at least one cationic acylpyridinium derivative of formula (I) according to claim 1 and at least 5 wt.%, based on the total weight of the agent, paraffin oil in coloring agents for keratin-containing fibers, in particular human hair, for color intensification in oxidative coloring.

## Revendications

**1.** Agent de coloration de fibres de kératine, **caractérisé en ce qu'**il contient, dans un support cosmétique,

(i) au moins un dérivé acylpyridinium cationique de la formule (I),

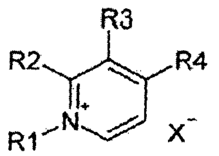

(I),

dans laquelle R1 représente un groupe alkyle en $C_1$ à $C_6$, un groupe alcényle en $C_2$ à $C_6$, un hydroxyalkyle en $C_1$ à $C_6$, un groupe alcoxy en $C_1$ à $C_6$-alkyle en $C_2$ à $C_6$, un groupe carboxy-alkyle en $C_2$ à $C_6$, un groupe aryle-alkyle en $C_1$ à $C_6$, un groupe hétéroaryl-alkyle en $C_1$ à $C_6$, un groupe aryle ou un groupe hétéroaryle,
R2, R3 et R4 représentent chacun indépendamment l'hydrogène, un groupe alkyle en $C_1$ à $C_6$, un atome d'halogène ou un groupe acyle en $C_1$ à $C_6$, à la condition qu'au moins un des radicaux R2, R3 et R4 soit un groupe acyle en $C_1$ à $C_6$,
$X^-$ est un anion physiologiquement acceptable,

(ii) au moins 5% en poids, par rapport au poids total de l'agent, d'huile de paraffine,
(iii) au moins du peroxyde d'hydrogène comme agent oxydant,
(iv) et au moins un composé colorant choisi parmi les précurseurs de colorants d'oxydation.

**2.** Agent selon la revendication 1, **caractérisé en ce qu'**il contient au moins un composé de la formule (I) qui est choisi parmi le p-toluènesulfonate de 4-acétyl-1-méthylpyridinium et le p-toluènesulfonate de 2-acétyl-1-méthylpyridinium.

**3.** Agent selon l'une des revendications 1 à 2, **caractérisé en ce que** les dérivés acylpyridinium de la formule (1) sont contenus dans une quantité de 0,1 à 10% en poids en particulier de 0,2 à 4% en poids, à chaque fois par rapport au poids total de l'agent.

**4.** Agent selon l'une des revendications 1 à 3, **caractérisé en ce qu'**il contient, dans un support cosmétique, comme premier composant au moins un composé choisi dans le groupe qui est formé p-toluène--sulfonate de 4-acétyl-1-méthylpyridinium, du benzène-sulfonate de 4-acétyl-1 méthylpyridinium, de l'hydrogène-sulfate de 4-acétyl-1-méthylpyridinium, l'acétate de 4-acétyl-1-méthylpyridinium, du p-toluène-sulfonate de 4-acétyl-1-allylpyridinium, du benzène-sulfonate d'4-acétyl-1-allylpyridinium, de l'hydrogène-sulfate de 4-acétyl-1-allylpyridinium, de l'acétate de 4-acétyl-1-allylpyridinium, du p-toluène-sulfonate de 2-acétyl-1-méthylpyridinium, du benzène-sulfonate de 2-acétyl-1-méthylpyridinium, de l'hydrogène-sulfate de 2-acétyl-1-methylpyridinium, de l'acétate de 2-acétyl-1-méthylpyridinium, du p-toluène-sulfonate de 2-acétyl-1-allylpyridinium, du benzène-sulfonate de 2-acétyl-1-allylpyridinium, de l'hydrogène-sulfate de 2-acétyl-1-allylpyridinium et l'acétate de 2-acétyl-1-allylpyridinium, comme deuxième composant au moins 10% en poids d'huile de paraffine, comme troisième composant du peroxyde d'hydrogène et

comme quatrième composant au moins un précurseur de colorant d'oxydation.

5. Agent selon l'une des revendications 1 à 3, **caractérisé en ce qu'**il contient entre 10 et 40% en poids, en particulier entre 15 et 30% en poids, par rapport au poids total de l'agent, d'huile de paraffine.

6. Procédé de production d'un moyen prêt à l'emploi de coloration de fibres de kératine, en particulier de cheveux humains, **caractérisé en ce que**, immédiatement avant l'application, on mélange entre eux un agent M1 contenant dans un support cosmétiquement acceptable

   (i) au moins un dérivé acylpyridinium cationique de la formule (I) selon la revendication 1,
   (ii) au moins 5% en poids, par rapport au poids total de l'agent, d'huile de paraffine,
   (iii) et au moins du peroxyde d'hydrogène comme agent oxydant,

   et un agent M2 contenant dans un support cosmétiquement acceptable

   (iv) au moins un composé colorant choisi parmi des précurseurs de colorants d'oxydation, et
   (v) au moins un agent d'alcalinisation,

   et **en ce que** l'agent prêt à l'emploi a une valeur de pH allant de pH 6 à pH 12, de préférence de pH 9,5 à pH 10,5.

7. Procédé selon la revendication 6, **caractérisé en ce que** l'agent M1 a un pH allant de pH 2 à pH 6.

8. Procédé selon la revendication 7, **caractérisé en ce que** l'agent M2 contient comme agent d'alcalinisation au moins de l'ammoniac.

9. Unité d'emballage de plusieurs composants (kit-of-parts) comprenant au moins deux récipients confectionnés séparément, un premier récipient C1 contenant au moins un agent M1 qui contient dans un support cosmétiquement acceptable

   (i) au moins un dérivé de acylpyridinium cationique de la formule (I) selon la revendication 1,
   (ii) au moins 5% en poids, par rapport au poids total de l'agent, d'huile de paraffine,
   (iii) et au moins du peroxyde d'hydrogène comme agent oxydant,

   et un second récipient C2 contenant au moins un agent M2 qui contient dans un support cosmétiquement acceptable

   (iv) au moins un composé colorant choisi parmi des précurseurs de colorants d'oxydation, et
   (v) au moins un agent d'alcalinisation.

10. Unité d'emballage de plusieurs composants (kit-of-parts) comprenant au moins deux récipients confectionnés séparément, un premier récipient CI contenant au moins un agent M1 qui contient dans un support cosmétiquement acceptable

   (i) au moins un dérivé acylpyridinium cationique de la formule (I) selon la revendication 1,
   (ii) au moins 5% en poids, par rapport au poids total de l'agent, d'huile de paraffine,
   (iii) et au moins du peroxyde d'hydrogène comme agent oxydant, l'agent ayant un pH M1 allant de pH 2 à pH 6,

   et un second récipient C2 contenant au moins un agent M2 qui contient dans un support cosmétiquement acceptable,

   (iv) (iv) au moins un composé colorant choisi parmi des précurseurs de colorants d'oxydation, et
   (v) (v) au moins un agent d'alcalinisation.

11. Unité d'emballage de plusieurs composants (kit-of-parts) comprenant au moins deux récipients confectionnés séparément, un premier récipient C1 contenant au moins un agent M1 qui contient dans un support cosmétiquement acceptable

   (i) au moins un dérivé acylpyridinium cationique de la formule (I) selon la revendication 1,
   (ii) au moins 5% en poids, par rapport au poids total de l'agent, d'huile de paraffine,
   (iii) et au moins du peroxyde d'hydrogène comme agent oxydant,

et un second récipient C2 contenant au moins un agent M2 qui contient dans un support cosmétiquement acceptable

(iv) au moins un composé colorant choisi parmi des précurseurs de colorants d'oxydation, et
(v) au moins un agent d'alcalinisation, l'agent d'alcalinisation contenu étant au moins de l'ammoniac.

**12.** Utilisation cosmétique d'une combinaison d'au moins un dérivé acylpyridinium cationique de la formule (1) selon la revendication 1 et au moins 5% en poids, par rapport au poids total de l'agent, d'huile de paraffine dans des colorants pour fibres de kératine, en particulier pour cheveux humains, pour intensifier la couleur lors de la coloration par oxydation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19745356
- DE 102007047685
- WO 2009135700 A1
- US 6371993 B1
- EP 998908 A2


### In der Beschreibung aufgeführte Nicht-Patentliteratur


- **B. KH. SCHRADER.** Grundlagen und Rezepturen der Kosmetika. Hüthig Buch Verlag, 1989